# EUROPEAN PATENT APPLICATION

(11) **EP 4 378 927 A1**
(43) Date of publication of application: **05.06.2024**
(21) Application number: 22849589.1
(22) Date of filing: 28.07.2022
(51) Int. Cl.: C07C 319/02, C07C 319/20, C07C 319/28, C07C 321/14, C07C 323/64, C08G 18/38, G02B 1/04

(54) **POLYTHIOL COMPOSITION AND APPLICATION OF SAME**

(30) Priority: 30.07.2021 JP 2021126033
(71) Applicant: Mitsui Chemicals, Inc., Tokyo 104-0028 (JP)
(72) Inventor: NAKANO, Shotaro, Omuta-shi, Fukuoka 836-8610 (JP); MURAKAMI, Masakazu, Omuta-shi, Fukuoka 836-8610 (JP)
(74) Representative: Mewburn Ellis LLP
(86) International application number: PCT/JP2022/029134
(87) International publication number: WO 2023/008528

(57) **Abstract**

A polythiol composition, comprising a polythiol compound, wherein: the polythiol composition comprises a compound C1 that has a retention time of from 32.0 minutes to 35.0 minutes in a high-performance liquid chromatography measurement performed under a specific measurement conditions A, and in the high-performance liquid chromatography measurement, the compound C1 has a peak area of 0.50 or more with respect to a total peak area of 100 of compounds contained in the polythiol composition.

## Description

### Technical Field

The present disclosure relates to a polythiol composition, and an application thereof.

### Background Art

Plastic lenses, which are resin-containing lenses, are lightweight, hardly breakable, and dyeable as compared to inorganic lenses; therefore, in recent years, the use of plastic lenses as eyeglass lenses, camera lenses, and the like has been rapidly increased.

For example, various studies have been conducted on thiourethane resin-containing lenses (see, for example, Patent Documents 1 to 3).
Patent Document 1: Japanese Patent Application Laid-Open (JP-A) No. S63-46213
Patent Document 2: JP-A No. H2-270859
Patent Document 3: JP-A No. H7-252207

### SUMMARY OF THE INVENTION

### Technical Problem

A thiourethane resin is usually produced using a polythiol composition and a polyisocyanate compound as raw materials.

For such a thiourethane resin, it may be demanded to further improve the heat resistance.

An object of one aspect of the disclosure is to provide: a polythiol composition from which a thiourethane resin having excellent heat resistance can be produced; and an application thereof.

### Solution to Problem

Means for solving the above-described problems encompass the following aspects.
<1> A polythiol composition, comprising a polythiol compound,
   wherein:
   the polythiol composition comprises a compound C1 that has a retention time of from 32.0 minutes to 35.0 minutes in a high-performance liquid chromatography measurement performed under the following measurement conditions A, and
   in the high-performance liquid chromatography measurement, the compound C1 has a peak area of 0.50 or more with respect to a total peak area of 100 of compounds contained in the polythiol composition:
      Measurement Conditions A
      YMC-Pack (registered trademark) ODS-A manufactured by YMC., Co., Ltd. (particle size S: 5 µm, pore diameter: 12 nm, column shape: Φ6 mm × 150 mm) is used as a column;
      a mixed solution of acetonitrile/0.01-mol/L aqueous potassium dihydrogen phosphate solution = 60/40 (vol/vol) is used as a mobile phase;
      a mixed solution of 160 mg of the polythiol composition and 10 mL of acetonitrile is used as a measurement solution;
      a UV detector having a measurement wavelength of 230 nm is used as a detector;
      the column temperature is set at 40°C;
      the flow rate is set at 1.0 mL/min; and
      the injection amount is set at 2 µL.
<2> The polythiol composition according to <1>, wherein the compound C1 has a molecular weight of 426.
<3> The polythiol composition according to <1> or <2>, wherein the compound C1 is a polythiol compound.
<4> The polythiol composition according to any one of <1> to <3>,
   wherein:
   the polythiol composition further comprises a compound C2 that has a retention time of from 16.0 minutes to 19.0 minutes in the high-performance liquid chromatography measurement, and
   in the high-performance liquid chromatography measurement, the compound C2 has a peak area of 1.39 or more with respect to a total peak area of 100 of compounds contained in the polythiol composition.
<5> The polythiol composition according to any one of <1> to <4>,
   wherein:
   the polythiol composition further comprises, as a main component, a polythiol compound (XA) comprising three or more mercapto groups,
   wherein:
      in the high-performance liquid chromatography measurement, a peak area of a compound (XB), the compound (XB) being obtained by substituting at least one of the three or more mercapto groups in the polythiol compound (XA) with a group represented by the following Formula (N1), is less than 1.10 with respect to a total peak area of 100 of compounds contained in the polythiol composition,
      and wherein:
         in the high-performance liquid chromatography measurement, a peak area of a compound (XC), the compound (XC) being obtained by substituting at least one of the three or more mercapto groups in the polythiol compound (XA) with a hydroxy group, is less than 2.40 with respect to a total peak area of 100 of compounds contained in the polythiol composition: 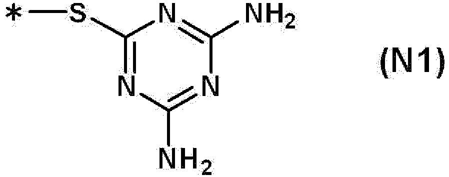
         wherein, in Formula (N1), * represents a binding position.
<6> The polythiol composition according to <5>, wherein the polythiol compound (XA) is at least one selected from the group consisting of 4-mercaptomethyl-1,8-dimercapto-3,6-dithiaoctane, 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.
<7> A polymerizable composition for an optical material, comprising:
   a polyiso(thio)cyanate compound; and
   the polythiol composition according to any one of <1> to <6>.
<8> The polymerizable composition for an optical material according to <7>, wherein the polyiso(thio)cyanate compound comprises at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.
<9> A resin, which is a cured product of the polymerizable composition for an optical material according to <7> or <8>.
<10> A formed body, comprising the resin according to <9>.
<11> An optical material, comprising the resin according to <9>.
<12> A lens, comprising the resin according to <9>.

### Advantageous Effects of Invention

According to one aspect of the disclosure, a polythiol composition from which a thiourethane resin having excellent heat resistance can be produced, and an application thereof are provided.

### DESCRIPTION OF EMBODIMENTS

In the disclosure, those numerical ranges that are expressed with "to" each denote a range that includes the numerical values stated before and after "to" as the lower limit value and the upper limit value, respectively.

In the disclosure, the term "step" encompasses not only a discrete step but also a step that cannot be clearly distinguished from other steps, as long as the intended purpose of the step is achieved.

In the disclosure, when there are plural substances that correspond to a component of a composition, the indicated amount of the component in the composition means, unless otherwise specified, a total amount of the plural substances existing in the composition.

In a set of numerical ranges that are stated in a stepwise manner in the disclosure, the upper limit value or the lower limit value of one numerical range may be replaced with the upper limit value or the lower limit value of other numerical range. Further, in a numerical range stated in the disclosure, the upper limit value or the lower limit value of the numerical range may be replaced with a relevant value indicated in any of Examples.

### [Polythiol Composition]

The polythiol composition of the disclosure is a polythiol composition containing a polythiol compound,
wherein:
the polythiol composition comprises a compound C1 that has a retention time of from 32.0 minutes to 35.0 minutes in a high-performance liquid chromatography measurement performed under the following measurement conditions A (hereinafter, also simply referred to as "high-performance liquid chromatography measurement"), and
in the high-performance liquid chromatography measurement, the compound C1 has a peak area of 0.50 or more with respect to a total peak area of 100 of compounds contained in the polythiol composition.

### (Measurement Conditions A)

YMC-Pack (registered trademark) ODS-A manufactured by YMC., Co., Ltd. (particle size S: 5 µm, pore diameter: 12 nm, column shape: Φ6 mm × 150 mm) is used as a column;
a mixed solution of acetonitrile/0.01-mol/L aqueous potassium dihydrogen phosphate solution = 60/40 (vol/vol) is used as a mobile phase;
a mixed solution of 160 mg of the polythiol composition and 10 mL of acetonitrile is used as a measurement solution;
a UV detector having a measurement wavelength of 230 nm is used as a detector;
the column temperature is set at 40°C;
the flow rate is set at 1.0 mL/min; and
the injection amount is set at 2 µL.

As the column, for example, YMC-Pack ODS-A (manufactured by YMC Co., Ltd.) can be used.

By using the polythiol composition of the disclosure as one of raw materials of a thiourethane resin, a thiourethane resin having excellent heat resistance can be produced.

### (Polythiol Composition)

The term "polythiol composition" used herein means a composition containing at least one polythiol compound.

In the disclosure, a polythiol compound contained in the polythiol composition is also referred to as "polythiol component".

The polythiol composition may also contain, as an impurity, a component other than the polythiol compound.

It is preferred that the polythiol composition contains at least one polythiol compound as a main component.

It is noted here that "the polythiol composition contains at least one polythiol compound as a main component" means that a total content of the at least one polythiol compound is not less than 50% with respect to a total amount of the polythiol composition.

The total content of the at least one polythiol compound with respect to the total amount of the polythiol composition is preferably not less than 60%, more preferably not less than 70%, still more preferably not less than 80%.

Similarly, in the disclosure, a feature that a composition contains a certain component (hereinafter, referred to as "component X") "as a main component" means that the content of the component X (when the component X consists of two or more compounds, a total content of the two or more compounds) is not less than 50% with respect to a total amount of the composition.

The content of the component X as a main component with respect to the total amount of the composition is preferably not less than 60%, more preferably not less than 70%, still more preferably not less than 80%.

The "%" in the description of the above-described expression "contain ... as a main component" means a ratio (% by area) of a total area of all peaks of the component X (e.g., at least one polythiol compound) with respect to a total area of all peaks of the composition (e.g., a polythiol composition), which ratio is determined by high-performance liquid chromatography under the above-described measurement conditions A.

The polythiol composition is, for example, a polythiol composition containing a known polythiol compound.

The polythiol compound is not particularly limited as long as it is a compound containing two or more thiol groups (other name: mercapto groups).

With regard to the polythiol compound, reference can be made as appropriate to the aforementioned known documents (i.e., JP-A No. S63-46213, JP-A No. H2-270859, JP-ANo. H7-252207, WO 2008/047626, and the like).

### (Compound C1)

The polythiol composition of the disclosure contains a compound C1.

The compound C1 is a compound that has a retention time of from 32.0 minutes to 35.0 minutes in a high-performance liquid chromatography measurement performed under the measurement conditions A.

In the polythiol composition of the disclosure, the compound C 1 may be contained singly, or in combination of two or more kinds thereof.

The compound C1 may or may not be a polythiol compound; however, the compound C1 is preferably a polythiol compound.

The compound C1 is a component effective for improving the heat resistance of a thiourethane resin produced using a polythiol composition.

In the polythiol composition of the disclosure, the peak area of the compound C1 with respect to a total peak area of 100 of compounds contained in the polythiol composition in the high-performance liquid chromatography measurement (hereinafter, also simply referred to as "peak area of the compound C1") is 0.50 or more, whereby an effect of improving the heat resistance of the resulting thiourethane resin is exerted.

From the standpoint of the hue of the resulting thiourethane resin, an upper limit of the peak area of the compound C1 is preferably 10.0, more preferably 5.0.

The compound C1 has a molecular weight of, for example, 426.

The term "molecular weight" used herein refers to a value measured by high-performance liquid chromatography mass spectrometry under the following measurement conditions B.

### (Measurement Conditions B)

YMC-Pack (registered trademark) C18RS manufactured by YMC., Co., Ltd. (particle size S: 3 µm, column shape: Φ4.6 mm × 250 mm) is used as a column,
a mixed solution of 10 mM aqueous ammonium acetate solution/acetonitrile = 30/70 (vol/vol) is used as a mobile phase,
a UV detector having a measurement wavelength of 230 nm is used as a detector,
the column temperature is set at 40°C,
the flow rate is set at 0.9 mL/min, and
the following conditions are applied as mass spectrometry conditions:
   Ionization Mode: ESI+
   Capillary Voltage: 3.0 kV
   Cone Voltage: 20 V
   Extractor: 4V
   Source Temp: 120°C
   Desolvation Temp: 400°C
   Cone Gas Flow: 50 L/Hr
   Desolvation Gas Flow: 800 L/Hr
   Mass: m/z 50-800

When the compound C1 has a molecular weight of 426 and is a polythiol compound, the compound C 1 preferably contains at least one selected from the group consisting of the following compounds (C1-1a), (C1-1b), (C1-1c), (C1-2a), (C1-2b), (C1-2c), and (C1-2d).

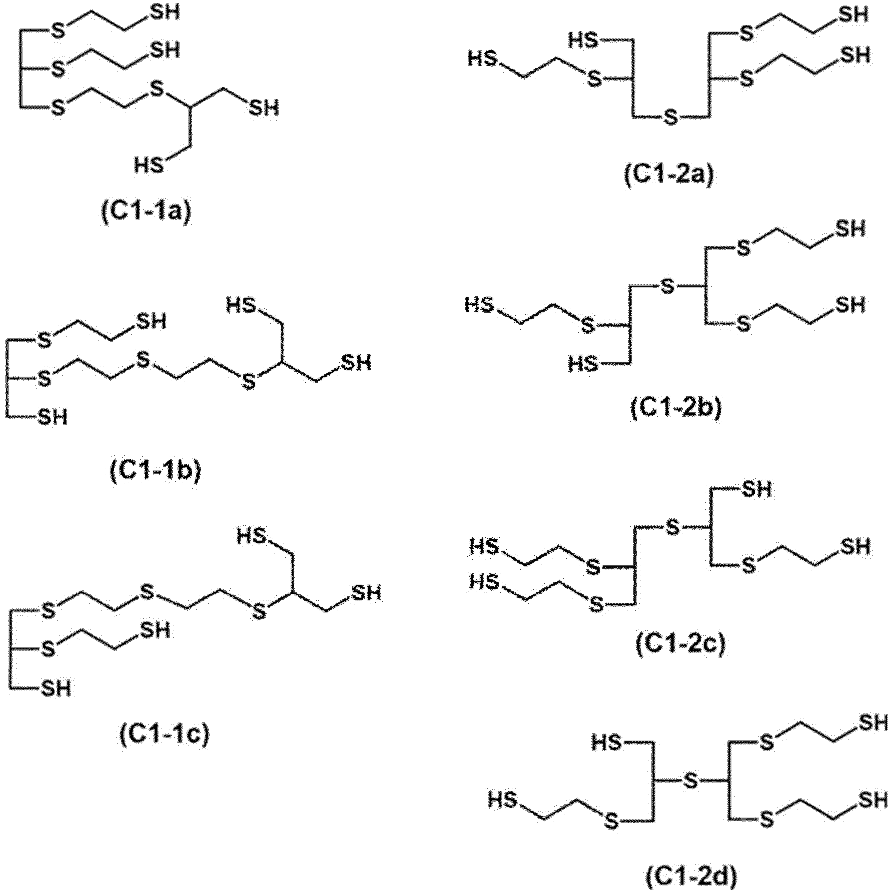

### (Compound C2)

The polythiol composition of the disclosure preferably further contains a compound C2.

By this, the heat resistance of the resulting thiourethane resin is further improved.

The compound C2 is a compound that has a retention time of from 16.0 minutes to 19.0 minutes in the high-performance liquid chromatography measurement performed under the measurement conditions A.

When the polythiol composition of the disclosure contains the compound C2, the compound C2 may be contained singly, or in combination of two or more kinds thereof.

The compound C2 may or may not be a polythiol compound.

In the high-performance liquid chromatography measurement performed under the measurement conditions A, the peak area of the compound C2 with respect to a total peak area of 100 of compounds contained in the polythiol composition (hereinafter, also simply referred to as "peak area of the compound C2") is preferably 1.39 or more.

By this, the heat resistance of the resulting thiourethane resin is further improved.

From the standpoint of the hue of the resulting thiourethane resin, an upper limit of the peak area of the compound C2 is preferably 10.0, more preferably 5.0.

### (Polythiol Compound (XA))

The polythiol composition of the disclosure preferably further contains, as a main component, a polythiol compound (XA) containing three or more mercapto groups.

The polythiol compound (XA) is a polythiol compound containing three or more mercapto groups. However, when the above-described compound C1 is a "polythiol compound containing three or more mercapto groups", the compound C1 is not included in the scope of the polythiol compound (XA). Similarly, when the polythiol composition of the disclosure contains the above-described compound C2 that is a "polythiol compound containing three or more mercapto groups", the compound C2 is not included in the scope of the polythiol compound (XA).

The polythiol compound (XA) is preferably at least one selected from the group consisting of:
4-mercaptomethyl-1,8-dimercapto-3,6-dithiaoctane,
4,8-dimercaptomethyl-1,11-dimercapto-3,6,9-trithiaundecane,
4,7-dimercaptomethyl-1,11 -dimercapto-3,6,9-trithiaundecane, and
5,7-dimercaptomethyl-1,11-dimercapto-3,6,9-trithiaundecane.

It is noted here that:
4-mercaptomethyl-1,8-dimercapto-3,6-dithiaoctane is a polythiol compound represented by the following Formula (1),
4,8-dimercaptomethyl-1,11-dimercapto-3,6,9-trithiaundecane is a polythiol compound represented by the following Formula (2),
4,7-dimercaptomethyl-1,11-dimercapto-3,6,9-trithiaundecane is a polythiol compound represented by the following Formula (3), and
5,7-dimercaptomethyl-1,11-dimercapto-3,6,9-trithiaundecane is a polythiol compound represented by the following Formula (4).

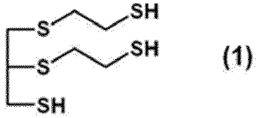

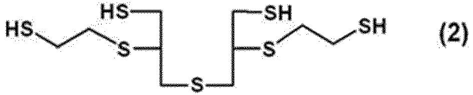

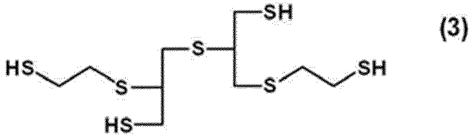

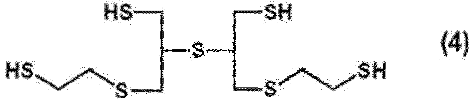

Examples of a more preferred aspect of the polythiol compound (XA) include:
an aspect of containing 4-mercaptomethyl-1,8-dimercapto-3,6-dithiaoctane (hereinafter, also referred to as "polythiol component A1") as a main component; and
an aspect of containing 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane (these three compounds are hereinafter also collectively referred to as "polythiol component A2") as a main component.
The polythiol composition of each aspect may also contain at least one other component (e.g., other polythiol compound and a component other than a polythiol compound) in addition to the main component.

In the aspect in which the polythiol compound (XA) contains the polythiol component A1 as a main component, from the standpoint of the heat resistance of the resulting thiourethane resin, the peak area of the compound C1 is 0.50 or more.

In the aspect in which the polythiol compound (XA) contains the polythiol component A1 as a main component, from the standpoint of further improving the heat resistance of the resulting thiourethane resin, the peak area of the compound C1 is preferably 1.00 or more, more preferably 1.20 or more.

In the aspect in which the polythiol compound (XA) contains the polythiol component A1 as a main component, from the standpoint of the hue of the resulting thiourethane resin, an upper limit of the peak area of the compound C1 is preferably 10.0, more preferably 5.0.

In the aspect in which the polythiol compound (XA) contains the polythiol component A1 as a main component, the compound C1 is preferably the above-described compound (C1-1).

Also in the aspect in which the polythiol compound (XA) contains the polythiol component A2 as a main component, from the standpoint of the heat resistance of the resulting thiourethane resin, the peak area of the compound C1 is 0.50 or more.

In the aspect in which the polythiol compound (XA) contains the polythiol component A2 as a main component, from the standpoint of further improving the heat resistance of the resulting thiourethane resin, the peak area of the compound C1 is preferably 2.30 or more, more preferably 2.50 or more, still more preferably 2.65 or more.

In the aspect in which the polythiol compound (XA) contains the polythiol component A2 as a main component, from the standpoint of the hue of the resulting thiourethane resin, an upper limit of the peak area of the compound C1 is preferably 10.0, more preferably 5.0.

In the aspect in which the polythiol compound (XA) contains the polythiol component A2 as a main component, the compound C1 is preferably at least one selected from the group consisting of the above-described compounds (C1-2a), (C1-2b), and (C1-2c).

In the aspect in which the polythiol compound (XA) contains the polythiol component A1 as a main component, from the standpoint of the heat resistance of the resulting thiourethane resin, the peak area of the compound C2 is preferably 1.39 or more.

In the aspect in which the polythiol compound (XA) contains the polythiol component A1 as a main component, from the standpoint of further improving the heat resistance of the resulting thiourethane resin, the peak area of the compound C2 is more preferably 2.40 or more, still more preferably 2.70 or more, yet still more preferably 2.90 or more.

In the aspect in which the polythiol compound (XA) contains the polythiol component A1 as a main component, from the standpoint of the hue of the resulting thiourethane resin, an upper limit of the peak area of the compound C2 is preferably 10.0, more preferably 5.0.

Also in the aspect in which the polythiol compound (XA) contains the polythiol component A2 as a main component, from the standpoint of the heat resistance of the resulting thiourethane resin, the peak area of the compound C2 is preferably 1.39 or more.

In the aspect in which the polythiol compound (XA) contains the polythiol component A2 as a main component, from the standpoint of further improving the heat resistance of the resulting thiourethane resin, the peak area of the compound C2 is more preferably 1.40 or more, still more preferably 1.41 or more.

In the aspect in which the polythiol compound (XA) contains the polythiol component A2 as a main component, from the standpoint of the hue of the resulting thiourethane resin, an upper limit of the peak area of the compound C2 is preferably 10.0, more preferably 5.0.

When the polythiol composition of the disclosure contains the polythiol compound (XA) as a main component, in the polythiol composition of the disclosure, it is preferred that:
in the high-performance liquid chromatography measurement, a peak area of a compound (XB), the compound (XB) being obtained by substituting at least one of the three or more mercapto groups in the polythiol compound (XA) with a group represented by the following Formula (N1), is less than 1.10 with respect to a total peak area of 100 of compounds contained in the polythiol composition, and
in the high-performance liquid chromatography measurement, a peak area of a compound (XC), the compound (XC) being obtained by substituting at least one of the three or more mercapto groups in the polythiol compound (XA) with a hydroxy group, is less than 2.40 with respect to a total peak area of 100 of compounds contained in the polythiol composition:

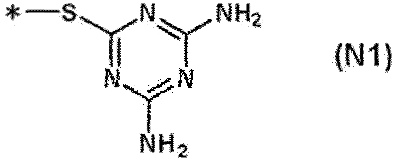

In Formula (N1), * represents a binding position.

The compound (XB) is a compound obtained by substituting at least one of the three or more mercapto groups in the polythiol compound (XA) with a group represented by Formula (N1).

The compound (XC) is a compound obtained by substituting at least one of the three or more mercapto groups in the polythiol compound (XA) with a hydroxy group.

The compound (XB) and the compound (XC) are both compounds that facilitate polymerization of the polythiol compound (XA) contained in the polythiol composition to cause an increase in the viscosity of the polythiol composition, and consequently shorten the pot life of the polythiol composition.

The polythiol composition of the disclosure preferably does not contain the compound (XB) or, even when the polythiol composition contains the compound (XB), a peak area of the compound (XB) in the high-performance liquid chromatography measurement is preferably less than 1.10 with respect to a total peak area of 100 of compounds contained in the polythiol composition.

When the polythiol compound (XA) is the compound (XB) of an aspect of containing the polythiol component A1 as a main component, the peak area of the compound (XB) is preferably less than 1.10, more preferably less than 0.50, still more preferably less than 0.25, yet still more preferably less than 0.10, with respect to a total peak area of 100 of compounds contained in the polythiol composition.

When the polythiol compound (XA) is the compound (XB) of an aspect of containing the polythiol component A2 as a main component, the peak area of the compound (XB) is preferably less than 1.10, more preferably less than 0.50, still more preferably less than 0.10, with respect to a total peak area of 100 of compounds contained in the polythiol composition.

The polythiol composition of the disclosure preferably does not contain the compound (XC) or, even when the polythiol composition contains the compound (XC), a peak area of the compound (XC) in the high-performance liquid chromatography measurement is preferably less than 2.40 with respect to a total peak area of 100 of compounds contained in the polythiol composition.

When the polythiol compound (XA) is the compound (XC) of an aspect of containing the polythiol component A1 as a main component, the peak area of the compound (XC) is preferably less than 2.40, more preferably less than 1.20, still more preferably less than 0.10, with respect to a total peak area of 100 of compounds contained in the polythiol composition.

When the polythiol compound (XA) is the compound (XC) of an aspect of containing the polythiol component A2 as a main component, the peak area of the compound (XC) is preferably less than 2.40, more preferably less than 2.00, still more preferably less than 1.00, yet still more preferably less than 0.10, with respect to a total peak area of 100 of compounds contained in the polythiol composition.

In the polythiol composition of the disclosure, when the content of the compounds (XB) and (XC) is limited in the above-described manner, unintended polymerization of the polythiol compound (XA) in the polythiol composition is suppressed, as a result of which an increase in viscosity is suppressed. Therefore, the polythiol composition according to this aspect has an excellent pot life (i.e., a long pot life).

The compound (XB) is a compound obtained by substituting at least one of the three or more mercapto groups in the polythiol compound (XA) with a group represented by the above-described Formula (N1).

Examples of the compound (XB) are shown below; however, the compound (XB) is not limited thereto.

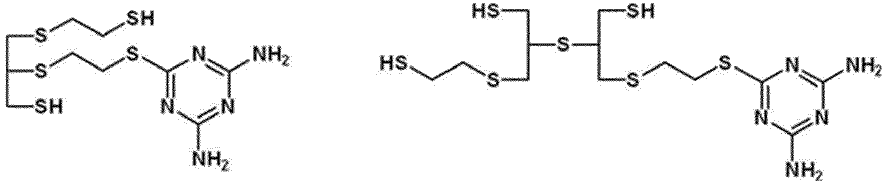

The compound (XC) is a compound obtained by substituting at least one of the three or more mercapto groups in the polythiol compound (XA) with a hydroxy group.

Examples of the compound (XC) are shown below; however, the compound (XC) is not limited thereto.

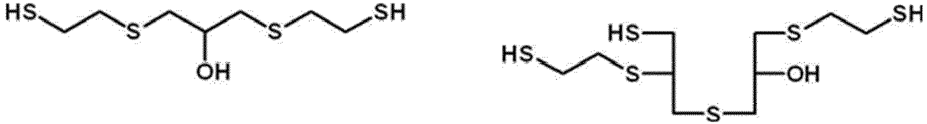

The polythiol composition of the disclosure may also contain other polythiol compound in addition to the above-described polythiol compounds.

Examples of the other polythiol compound include methanedithiol, 1,2-ethanedithiol, 1,2,3-propanetrithiol, pentaerythritol tetrakis(2-mercaptoacetate), pentaerythritol tetrakis(3-mercaptopropionate), bis(mercaptoethyl) sulfide, 2,5-dimercaptomethyl-1,4-dithiane, tetrakis(mercaptomethylthiomethyl)methane, tetrakis(2-mercaptoethylthiomethyl)methane, tetrakis(3-mercaptopropylthiomethyl)methane, bis(2,3-dimercaptopropyl) sulfide, 2,5-dimercaptomethyl-1,4-dithiane, 2,5-dimercapto-1,4-dithiane, 2,5-dimercaptomethyl-2,5-dimethyl-1,4-dithiane, 1,1,3,3-tetrakis(mercaptomethylthio)propane, 1,1,2,2-tetrakis(mercaptomethylthio)ethane, and 4,6-bis(mercaptomethylthio)-1,3-dithiane.

### [Method of Producing Polythiol Composition (Production Method A)]

The above-described polythiol composition of the disclosure is produced by, for example, the below-described production method (hereinafter, also referred to as "production method A").

The production method A is a method of producing a polythiol composition, which method includes a step of generating the polythiol composition of the disclosure by reacting a thiourethane resin and an active hydrogen compound with each other.

According to the production method A, the polythiol composition of the disclosure can be produced using a thiourethane resin as a starting material.

In the production method A, it is believed that a thiourethane resin and an active hydrogen compound react with each other, and thereby the thiourethane resin is chemically decomposed, as a result of which the polythiol composition of the disclosure is obtained as a decomposition product of the thiourethane resin.

### (Thiourethane Resin)

The thiourethane resin is a starting material in the production method A.

The thiourethane resin is not particularly limited, and examples thereof include those thiourethane resins that are described in known documents, such as JP-A No. S63-46213, JP-ANo. H2-270859, JP-ANo. H7-252207, and WO 2008/047626.

The thiourethane resin is usually produced as a reaction product of a polyisocyanate compound and a polythiol composition that are used as raw materials.

Examples of the thiourethane resin include:
thiourethane resins obtained from MR-6, MR-7, MR-8, MR-8 Plus, MR-60, MR-10, and MR-20 (all of which are manufactured by Mitsui Chemicals, Inc.), which are high-refractive-index lens materials; and
EYAS 1.60 (manufactured by HOYA Corporation).

The thiourethane resin is preferably one collected in at least one of an eyeglass lens production process, an eyeglass production process, or an eyeglass disposal process. According to this aspect, recycle of the thiourethane resin that is an eyeglass lens material is realized.

It is noted here that:
the "eyeglass lens production process" means a process of producing a resin by mixing and cast-polymerizing monomers that are raw materials of the resin, and/or a process of obtaining an eyeglass lens by cutting a resin formed body;
the "eyeglass production process" means a process of producing an eyeglass by combining eyeglass lenses and other members such as an eyeglass frame with each other; and
the "eyeglass disposal process" means a process of disposing of, for example, an eyeglass that has been produced but is no longer needed, or a used eyeglass.

In all of these processes, a thiourethane resin, which is an eyeglass lens material, may be generated as a waste.

In the present aspect, a thiourethane resin generated in at least one of these processes is used as a starting material, and this thiourethane resin and an active hydrogen compound are reacted with each other to obtain the polythiol composition of the disclosure as a decomposition product of the thiourethane resin.

As described above, in the production method A, the use of a used thiourethane resin for the production of a polythiol composition enables to reduce the amount of the thiourethane resin to be disposed of by incineration, as a result of which the generation of greenhouse gasses such as carbon dioxide, nitrogen monoxide, and sulfur dioxide, as well as the generation of air pollutants such as sulfur oxide and nitrogen oxide, can be reduced. In addition, since thiourea is not used for the production of a polythiol compound, no thiourea-containing waste water is generated; therefore, the production method A is an environmentally-friendly production method.

As described above, the production method A is effective as a method of recycling a thiourethane resin.

By the production method A, the polythiol composition of the disclosure that contains the compound C1 is obtained. This point is effective in terms of ensuring the material traceability.

The above-described starting material preferably contains a cut swarf (e.g., cut powder and/or cut pieces; the same applies hereinafter) containing a thiourethane resin.

In the step of generating the polythiol composition according to this aspect, a cut swarf containing a thiourethane resin and an active hydrogen compound are brought into contact with each other, and the thiourethane resin and the active hydrogen compound are thereby reacted with each other.

In this aspect, because of superior reactivity between the active hydrogen compound and the thiourethane resin contained in the starting material, the polythiol composition can be generated more effectively.

### (Active Hydrogen Compound)

The active hydrogen compound functions as a decomposition agent against the thiourethane resin that is a starting material.

From the standpoint of this function, the active hydrogen compound is preferably at least one selected from the group consisting of an amine compound and an alcohol compound.

### -Amine Compound-

The amine compound is preferably an amine compound which contains at least one of an amino group or a monoalkylamino group, and has a total number of amino groups and monoalkylamino groups of from 1 to 6 (preferably from 1 to 3, more preferably 1 or 2).

From the standpoint of further improving the reactivity with the thiourethane resin, the molecular weight of the amine compound is preferably 1,000 or less, more preferably 500 or less, still more preferably 300 or less, yet still more preferably 200 or less.

A lower limit of the molecular weight of the amine compound is, for example, not less than 45, preferably not less than 59, more preferably not less than 60.

One preferred example of the amine compound is an amine compound of 300 or less in molecular weight, which contains at least one of an amino group or a monoalkylamino group, and has a total number of amino groups and monoalkylamino groups of 1 or 2.

Specific examples of the amine compound include alkylamine having from 2 to 10 carbon atoms, aralkylamine having from 7 to 10 carbon atoms (e.g., benzylamine), dialkylamine having from 2 to 10 carbon atoms (e.g., di-n-butylamine), alkyldiamine having from 2 to 10 carbon atoms (e.g., ethylenediamine and bis(2-aminoethyl)ether), alkyltriamine having from 2 to 10 carbon atoms (e.g., bis(2-aminoethyl)amine), hydroxyalkylamine having from 2 to 10 carbon atoms (e.g., monoethanolamine), bis(hydroxyalkyl)amine having from 2 to 10 carbon atoms (e.g., bis(hydroxyethyl)amine), cyclic amine having from 2 to 10 carbon atoms (e.g., morpholine), and secondary amine such as alkyl(hydroxyalkyl)amine having from 2 to 10 carbon atoms (e.g., methylethanolamine and isopropylethanolamine).

The amine compound is preferably benzylamine, di-n-butylamine, ethylenediamine, or monoethanolamine.

### -Alcohol Compound-

The alcohol compound may be a monoalcohol compound containing only one hydroxy group, or a polyol compound having two or more hydroxy groups.

From the standpoint of further improving the reactivity with the thiourethane resin, the molecular weight of the alcohol compound is preferably 1,000 or less, more preferably 500 or less, still more preferably 300 or less, yet still more preferably 200 or less.

A lower limit of the molecular weight of the alcohol compound is, for example, not less than 40, preferably not less than 50, more preferably not less than 60.

The alcohol compound preferably contains an alcohol compound having a boiling point of from 135°C to 250°C (hereinafter, also referred to as "alcohol compound A").

The term "boiling point" used herein means a boiling point under 1 atm (101,325 Pa).

A ratio of the alcohol compound A with respect to a total amount of the alcohol compound is preferably from 50% by mass to 100% by mass, more preferably from 60% by mass to 100% by mass, still more preferably from 80% by mass to 100% by mass.

The alcohol compound is preferably benzyl alcohol, phenethyl alcohol, 2-octanol, 2-ethyl-1-hexanol, 1-decanol, 1-nonanol, 1-octanol, 1-heptanol, 1-hexanol, 1-pentanol, propylene glycol, or ethylene glycol, more preferably benzyl alcohol, phenethyl alcohol, 1-decanol, 1-nonanol, 1-octanol, 1-heptanol, 1-hexanol, 1-pentanol, propylene glycol, or ethylene glycol, still more preferably benzyl alcohol, phenethyl alcohol, 2-octanol, 1-octanol, 1-heptanol, 1-hexanol, 1-pentanol, or propylene glycol.

### (Reaction Solvent)

In the step of generating the polythiol composition, the thiourethane resin and the active hydrogen compound are preferably reacted with each other in the presence of a reaction solvent.

The reaction solvent is preferably a hydrocarbon compound having from 5 to 12 (preferably from 6 to 10, more preferably from 7 to 9) carbon atoms, an ether compound having from 4 to 12 carbon atoms, a ketone compound having from 3 to 12 carbon atoms, an ester compound having from 4 to 12 carbon atoms, an alcohol compound having from 2 to 12 carbon atoms, or a nitrile compound having from 2 to 12 carbon atoms.

The above-described hydrocarbon compound is preferably hexane, heptane, octane, nonane, decane, xylene, mesitylene, or toluene, more preferably heptane, octane, nonane, xylene, mesitylene, or toluene, particularly preferably xylene or toluene.

The above-described ether compound is preferably diethyl ether, tetrahydrofuran, 2-methyltetrahydrofuran, dimethoxyethane, or 1,4-dioxane, particularly preferably dimethoxyethane.

The above-described ketone compound is preferably acetone, methyl ethyl ketone, methyl isobutyl ketone, or 2-octanone, particularly preferably methyl isobutyl ketone.

The above-described ester compound is preferably ethyl acetate, butyl acetate, or pentyl acetate, particularly preferably pentyl acetate.

The above-described alcohol compound is preferably ethanol, 1-propanol, isopropanol, 1-butanol, 1-pentanol, 1-octanol, 2-octanol, benzyl alcohol, phenethyl alcohol, ethylene glycol, propylene glycol, or methyl cellosolve, particularly preferably benzyl alcohol or ethylene glycol.

The above-described nitrile compound is preferably acetonitrile or propionitrile, particularly preferably acetonitrile.

As the reaction solvent, these compounds may be used singly, or in combination of two or more kinds thereof.

### (Preferred Aspect of Step of Generating Polythiol Composition)

The step of generating the polythiol composition is preferably a step of reacting a thiourethane resin and an active hydrogen compound with each other to generate a polythiol composition and a polyamine compound.

In this preferred aspect, a decomposition reaction occurs in which the thiourethane resin is decomposed into the polythiol composition and the polyamine compound by the active hydrogen compound functioning as a decomposition agent.

When the active hydrogen compound is an amine compound, the decomposition reaction is an aminolysis reaction, whereas when the active hydrogen compound is an alcohol compound, the decomposition reaction is an alcoholysis reaction.

### (Resin Mixture Containing Thiourethane Resin)

The step of generating the polythiol composition may include contacting the active hydrogen compound and a resin mixture containing the thiourethane resin with each other to cause the active hydrogen compound and the thiourethane resin in the resin mixture to react with each other.

The resin mixture containing a thiourethane resin further contains components other than the thiourethane resin.

Examples of the components other than the thiourethane resin include a resin other than the thiourethane resin, and an inorganic material (e.g., glass) for lens production.

The resin other than the thiourethane resin is not particularly limited.

The scope of the resin mixture containing a thiourethane resin and a resin other than the thiourethane resin includes, for example:
a hybrid material of a thiourethane resin and a urethane resin, which is produced by adding a polyol compound to a raw material in the production of the thiourethane resin; and
a hybrid material of a thiourethane resin and a urea resin, which is produced by adding a polyamine compound to a raw material in the production of the thiourethane resin.

Examples of the resin other than the thiourethane resin include:
a polyolefin film that protects the surface of a resin formed body for eyeglass lens production;
a hard coat or a primer coat that protects the surface of a resin formed body for eyeglass lens production;
an abrasive agent used for grinding a resin formed body for eyeglass lens production;
a resin material used for immobilizing a resin formed body for eyeglass lens production during cutting of the resin formed body; and
a tape or a tape glue that is used for immobilizing a glass mold used in the preparation of a resin formed body for eyeglass lens production.

The resin mixture preferably contains, as a resin other than the thiourethane resin, at least one selected from the group consisting of a polycarbonate resin, a polyallylcarbonate resin, an acrylic resin, a urethane resin, and an episulfide resin.

These resins, like thiourethane resins, can also be used as materials for eyeglass lenses.

The resin mixture containing a thiourethane resin is preferably one collected in at least one of an eyeglass lens production process, an eyeglass production process, or an eyeglass disposal process.

The meaning of each of the eyeglass lens production process, the eyeglass production process, and the eyeglass disposal process is as described above.

The resin mixture containing a thiourethane resin preferably contains a cut swarf containing the thiourethane resin.

### (Reaction Mixture Containing Polythiol Composition)

The step of generating the polythiol composition may also be a step of obtaining a reaction mixture containing a polythiol composition as a target material by reacting a thiourethane resin and an active hydrogen compound with each other, thereby generating the polythiol composition of the disclosure.

Examples of components other than the polythiol composition in the reaction mixture include the above-described reaction solvent, residue of the raw material(s) (thiourethane resin and/or active hydrogen compound), and impurities contained in the raw materials.

### (Separation Step)

The production method A may also include the separation step of separating the polythiol composition as a target material from the above-described reaction mixture containing the polythiol composition.

A separation method in the separation step is not particularly limited, and any known method can be applied.

Examples of the separation method in the separation step include filtration, decantation, extraction, distillation, drying (including vacuum drying), and purification (e.g., column chromatography). These separation methods may be used in combination of two or more thereof.

Examples of a method of separating the polythiol composition include a method of extracting the polythiol composition with an organic solvent or inorganic solvent that can dissolve a polythiol compound.

As a method of purifying the polythiol composition, a general purification method such as column purification, distillation purification, recrystallization purification, or salt extraction is employed.

When the step of generating the polythiol composition is the above-described step of generating a polythiol composition and a polyamine compound, the separation step preferably includes at least one of obtaining a filtrate containing the polythiol composition as a filtrate or obtaining a mixture containing a polyurea compound that is a polyamine derivative as a filtration residue, by filtration of a reaction mixture containing the polythiol composition and the polyamine derivative.

When the separation step includes obtaining a filtrate containing the polythiol composition as a filtrate, the polythiol composition can be obtained by separating the polythiol composition from the filtrate.

In this case, one example of the separation step is a method that includes:
filtering a reaction mixture containing a polythiol composition and a polyamine compound to obtain a filtrate containing the polythiol composition;
adding an alkali metal-containing base and then water to the filtrate containing the polythiol composition to perform extraction and thereby obtain a water extract containing an alkali metal salt of the polythiol composition;
adding an acid to the water extract containing an alkali metal salt of the polythiol composition to obtain an aqueous liquid containing the polythiol composition;
adding a hydrocarbon compound having from 5 to 12 carbon atoms as an extraction solvent to the aqueous liquid containing the polythiol composition to perform extraction and thereby obtain an extract containing the polythiol composition; and
separating the polythiol composition from the extract containing the polythiol composition.

In this example, first, the polythiol composition in the filtrate containing the polythiol composition is converted to an alkali metal salt and subsequently extracted with water to obtain a water extract containing the alkali metal salt of the polythiol composition. Next, an acid is added to this water extract to convert the alkali metal salt of the polythiol composition back to the polythiol composition. From the resulting aqueous liquid containing the polythiol composition, the polythiol composition is extracted with the above-described extraction solvent to obtain an extract containing the polythiol composition. The polythiol composition is then separated from the thus obtained extract containing the polythiol composition.

According to this example, even when the filtrate containing the polythiol composition contains a large amount of components other than the polythiol composition, a polythiol composition having a higher purity of a polythiol component as a main component can be obtained.

In the above-described example, the alkali metal in the alkali metal-containing base is preferably sodium, potassium, or lithium, more preferably sodium or potassium.

Examples of the alkali metal-containing base include sodium methoxide, sodium ethoxide, sodium propoxide, sodium hydroxide, potassium hydroxide, and lithium hydroxide.

If necessary, the alkali metal-containing base can be added to the filtrate in the form of an alcohol solution (e.g., a methanol solution or an ethanol solution).

In the above-described example, examples of the acid added to the water extract containing an alkali metal salt of the polythiol composition include hydrochloric acid, carbonic acid, nitric acid, sulfuric acid, acetic acid, formic acid, and oxalic acid.

In the above-described example, the extraction solvent may be used singly, or in combination of two or more kinds thereof.

In a separation aspect B, a preferred aspect of the extraction solvent is the same as that of the above-described reaction solvent.

It is noted here, however, that the reaction solvent and the extraction solvent may be the same or different.

When the separation step includes obtaining a mixture containing a polyurea compound as a filtration residue or as a residue obtained by decantation or extraction, an active hydrogen compound (preferably an amine compound and/or an alcohol compound) is brought into contact with the mixture containing the polyurea compound as the filtration residue or the residue to cause the polyurea compound in the mixture and the active hydrogen compound to react with each other, whereby a polyamine compound can be generated as a thiourethane resin raw material. By separating the polyamine compound from the resulting reaction mixture containing the polyamine compound, the polyamine compound as a thiourethane resin raw material can be obtained.

### (Other Steps)

The production method A, if necessary, may include other steps in addition to the above-described steps.

Examples of the other steps include:
the sieving step of applying the thiourethane resin to a sieve prior to the step of generating the polythiol composition;
the step of washing the thiourethane resin prior to the step of generating the polythiol composition; and
the step of crushing and/or pulverizing the thiourethane resin prior to the step of generating the polythiol composition.

Specific examples of these other steps will now be described.

The production method A may include, prior to the step of generating the polythiol composition, the classification step of classifying a cut swarf containing the thiourethane resin to obtain a powder containing the thiourethane resin and having a smaller average particle size than the cut swarf.

When the production method A includes this classification step, the step of generating the polythiol composition includes reacting the thiourethane resin in the obtained powder and the active hydrogen compound with each other by contacting the obtained powder and the active hydrogen compound with each other.

### Examples of a classification method include sieving and centrifugation.

With regard to an aspect in which classification is performed by sieving, reference can be made to the below-described sieving step.

The production method A may also include, prior to the step of generating the polythiol composition, the sieving step of sieving a cut powder containing the thiourethane resin to obtain a powder containing the thiourethane resin and having passed through a sieve.

When the production method A includes this sieving step, the step of generating the polythiol composition includes reacting the thiourethane resin in the obtained powder and the active hydrogen compound with each other by contacting the obtained powder and the active hydrogen compound with each other.

The above-described sieve is not particularly limited.

The nominal mesh size of the sieve, which is defined by JIS Z-8801-1:2019, is, for example, from 0.1 mm to 20 mm, preferably from 0.1 mm to 10 mm, more preferably from 0.1 mm to 5 mm, still more preferably from 0.1 mm to 2 mm, yet still more preferably from 0.3 mm to 2 mm, further more preferably from 0.5 mm to 1.5 mm.

The method of producing a polythiol composition according to the production method A may also include, prior to the step of generating the polythiol composition, the washing step of washing the powder containing the thiourethane resin using a hydrocarbon compound having from 5 to 12 carbon atoms as a washing solvent.

When the method of producing a polythiol composition according to the production method A includes this washing step, the step of generating the polythiol composition includes reacting the thiourethane resin in the powder washed in the washing step and the active hydrogen compound with each other by contacting the powder and the active hydrogen compound with each other.

The hydrocarbon compound as the washing solvent may be used singly, or in combination of two or more kinds thereof.

A preferred aspect of the hydrocarbon compound used as the washing solvent is the same as that of the hydrocarbon compound used as the above-described reaction solvent.

It is noted here, however, that the reaction solvent and the washing solvent may be the same or different.

A washing method in the washing step is not particularly limited, and any known method, such as a method of adding the above-described washing solvent to the thiourethane resin powder and mixing the resultant, can be applied.

When the production method A includes the above-described sieving step and washing step, the sieving step and the washing step are preferably performed in this order. In this case, since it is not necessary to wash the cut powder not passing through the sieve, the amount of the washing solvent to be used can be further reduced.

### [Polymerizable Composition for Optical Material]

The polymerizable composition for an optical material according to the disclosure (hereinafter, also referred to as "the polymerizable composition of the disclosure") contains:
a polyiso(thio)cyanate compound; and
the above-described polythiol composition of the disclosure.

The polymerizable composition of the disclosure contains the polythiol composition of the disclosure and, therefore, exerts the same effects as those of the polythiol composition of the disclosure.

### (Polyiso(thio)cyanate Compound)

The polyiso(thio)cyanate compound is not particularly limited as long as it is a compound having at least two iso(thio)cyanate groups in one molecule.

Specific examples of the polyiso(thio)cyanate compound include:
aliphatic polyisocyanate compounds, such as tetramethylene diisocyanate, pentamethylene diisocyanate, hexamethylene diisocyanate, heptamethylene diisocyanate, octamethylene diisocyanate, 2,2,4-trimethylhexamethylene diisocyanate, 2,4,4-trimethylhexamethylene diisocyanate, lysine diisocyanate methyl ester, lysine triisocyanate, and xylylene diisocyanate;
alicyclic polyisocyanate compounds, such as isophorone diisocyanate, bis(isocyanatomethyl)cyclohexane, bis(isocyanatocyclohexyl)methane, dicyclohexyldimethylmethane diisocyanate, 2,5-bis(isocyanatomethyl)bicyclo-[2.2.1]-heptane, 2,6-bis(isocyanatomethyl)bicyclo-[2.2.1]-heptane, 3,8-bis(isocyanatomethyl)tricyclodecane, 3,9-bis(isocyanatomethyl)tricyclodecane, 4,8-bis(isocyanatomethyl)tricyclodecane, and 4,9-bis(isocyanatomethyl)tricyclodecane;
aromatic polyisocyanate compounds, such as tolylene diisocyanate, 4,4'-diphenylmethane diisocyanate, diphenyl sulfide-4,4-diisocyanate, and phenylene diisocyanate;
heterocyclic polyisocyanate compounds, such as 2,5-diisocyanatothiophene, 2,5-bis(isocyanatomethyl)thiophene, 2,5-diisocyanatotetrahydrothiophene, 2,5-bis(isocyanatomethyl)tetrahydrothiophene, 3,4-bis(isocyanatomethyl)tetrahydrothiophene, 2,5-diisocyanato-1,4-dithiane, 2,5-bis(isocyanatomethyl)-1,4-dithiane, 4,5-diisocyanato-1,3-dithiolane, and 4,5-bis(isocyanatomethyl)-1,3-dithiolane;
aliphatic polyisothiocyanate compounds, such as hexamethylene diisothiocyanate, lysine diisothiocyanate methyl ester, lysine triisothiocyanate, and xylylene diisothiocyanate;
alicyclic polyisothiocyanate compounds, such as isophorone diisothiocyanate, bis(isothiocyanatomethyl)cyclohexane, bis(isothiocyanatocyclohexyl)methane, cyclohexane diisothiocyanate, methylcyclohexane diisothiocyanate, 2,5-bis(isothiocyanatomethyl)bicyclo-[2.2.1]-heptane, 2,6-bis(isothiocyanatomethyl)bicyclo-[2.2.1]-heptane, 3,8-bis(isothiocyanatomethyl)tricyclodecane, 3,9-bis(isothiocyanatomethyl)tricyclodecane, 4,8-bis(isothiocyanatomethyl)tricyclodecane, and 4,9-bis(isothiocyanatomethyl)tricyclodecane;
aromatic polyisothiocyanate compounds, such as tolylene diisothiocyanate, 4,4-diphenylmethane diisothiocyanate, and diphenyl disulfide-4,4-diisothiocyanate; and
sulfur-containing heterocyclic polyisothiocyanate compounds, such as 2,5-diisothiocyanatothiophene, 2,5-bis(isothiocyanatomethyl)thiophene, 2,5-isothiocyanatotetrahydrothiophene, 2,5-bis(isothiocyanatomethyl)tetrahydrothiophene, 3,4-bis(isothiocyanatomethyl)tetrahydrothiophene, 2,5-diisothiocyanato-1,4-dithiane, 2,5-bis(isothiocyanatomethyl)-1,4-dithiane, 4,5-diisothiocyanato-1,3-dithiolane, and 4,5-bis(isothiocyanatomethyl)-1,3-dithiolane.

The polyiso(thio)cyanate compound may contain at least one selected from the above-exemplified compounds.

As the polyiso(thio)cyanate compound, for example, a halogen substitute such as a bromine substitute or a chlorine substitute, an alkyl substitute, an alkoxy substitute, a nitro substitute, a prepolymer-type product modified with a polyhydric alcohol, a carbodiimide-modified product, a urea-modified product, a burette-modified product, or a dimerization or trimerization reaction product of any of the above-exemplified compounds can be used as well.

The polyiso(thio)cyanate compound is preferably a polyisocyanate compound, and preferably contains at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.

A mixing ratio of the polythiol composition and the polyiso(thio)cyanate compound is not particularly limited and, for example, a molar ratio between the mercapto groups of the polythiol compound contained in the polythiol composition and the iso(thio)cyanate groups of the polyiso(thio)cyanate (mercapto groups/iso(thio)cyanate groups) is preferably from 0.5 to 3.0, more preferably from 0.6 to 2.0, still more preferably from 0.8 to 1.3. When the mixing ratio is in the above-described range, various performance required for a plastic lens or the like, such as refractive index and heat resistance, tend to be satisfied in a well-balanced manner.

The polymerizable composition of the disclosure may also contain other components in addition to the polythiol composition and the polyiso(thio)cyanate compound.

Examples of the other components include a polymerization catalyst, an internal mold release agent, a resin modifier, a chain extender, a crosslinking agent, a radical scavenger, a light stabilizer, a UV absorber, an antioxidant, an oil-soluble dye, a filler, an adhesion improver, an antimicrobial agent, an antistatic agent, a dye, a fluorescent brightener, a fluorescent pigment, and a blue ink agent such as an inorganic pigment.

Examples of the polymerization catalyst include tertiary amine compounds, inorganic and organic acid salts thereof, metal compounds, quaternary ammonium salts, and organic sulfonic acids.

As the internal mold release agent, an acidic phosphoric acid ester can be used. Examples of the acidic phosphoric acid ester include phosphoric acid monoesters and phosphoric acid diesters, and these may be used singly, or in combination of two or more kinds thereof.

Examples of the resin modifier include episulfide compounds, alcohol compounds, amine compounds, epoxy compounds, organic acids and anhydrides thereof, and olefin compounds containing a (meth)acrylate compound or the like.

The polymerizable composition of the disclosure can be obtained by mixing the above-described components.

### [Resin]

The resin of the disclosure is a cured product of the above-described polymerizable composition of the disclosure.

The resin of the disclosure is obtained by curing the above-described polymerizable composition of the disclosure.

The polymerizable composition can be cured by polymerizing the monomers contained in the polymerizable composition (specifically the polythiol composition and the polyiso(thio)cyanate compound; the same applies hereinafter). As a pretreatment of this polymerization, the polymerizable composition may be subjected to filtration, degassing, and the like.

The conditions for the polymerization of the monomers contained in the polymerizable composition (e.g., polymerization temperature and polymerization time) are set as appropriate, taking into consideration the formulation of the composition, the types and the amounts of the monomers used in the composition, the type and the amount of a polymerization catalyst used in the composition, and the properties of the below-described mold if used, and the like.

The polymerization temperature is, for example, from -50°C to 150°C, or from 10°C to 150°C.

The polymerization time is, for example, from 1 hour to 200 hours, or from 1 hour to 80 hours.

The resin of the disclosure may also be obtained by performing a treatment such as annealing on a polymer obtained by the polymerization of the monomers.

The temperature of this annealing is, for example, from 50°C to 150°C, from 90°C to 140°C, or from 100°C to 130°C.

The formed body of the disclosure contains the resin of the disclosure.

The formed body of the disclosure is obtained by curing the polymerizable composition of the disclosure in the same manner as the resin of the disclosure.

Preferred conditions for the curing of the polymerizable composition, i.e., the polymerization of the monomers contained in the polymerizable composition, are as described above.

One example of a preferred method of producing the formed body of the disclosure is cast polymerization.

In the cast polymerization, first, the above-described polymerizable composition is injected between casting molds held by a gasket, a tape, or the like. In this process, if necessary, a defoaming treatment, a filtration treatment, and the like may be performed.

Next, the monomers contained in the polymerizable composition injected between the casting molds are polymerized to cure the composition between the casting molds and thereby obtain a cured product. Subsequently, the cured product is removed from the casting molds to obtain a resin-containing formed body.

The polymerization of the monomers may also be performed by heating the polymerizable composition. This heating can be performed using, for example, a heating apparatus equipped with a mechanism for heating a material to be heated in an oven, water, or the like.

In the cast polymerization, formed bodies of various shapes (e.g., the below-described optical material) can be obtained by modifying the shape of the casting molds.

### [Optical Material]

The optical material of the disclosure contains the resin of the disclosure.

The optical material of the disclosure is obtained by curing the polymerizable composition of the disclosure in the same manner as the resin of the disclosure.

Preferred conditions for the curing of the polymerizable composition, i.e., the polymerization of the monomers contained in the polymerizable composition, are as described above.

Examples of the optical material include lenses (e.g., eyeglass lenses, camera lenses, and polarizing lenses), and light-emitting diodes (LEDs).

The optical material of the disclosure may include a coating layer formed on one side or both sides of the resin of the disclosure (or a formed body containing the resin of the disclosure).

Specific examples of the coating layer include a primer layer, a hard coat layer, an anti-reflection layer, an anti-fog coating layer, an anti-fouling layer, and a water-repellent layer.

These coating layers may each be formed singly, or plural coating layers may be formed in the form of a multilayer structure. When coating layers are formed on both sides, the same coating layer may be formed on the respective sides, or different coating layers may be formed on the respective sides.

Components of the coating layer can be selected as appropriate in accordance with the intended purpose.

Examples of the components of the coating layer include a resin (e.g., a urethane resin, an epoxy resin, a polyester resin, a melamine resin, or a polyvinyl acetal resin), an infrared absorber, a light stabilizer, an antioxidant, a photochromic compound, a dye, a pigment, and an antistatic agent.

With regard to the eyeglass lens and the coating layer, reference can be made as appropriate to the descriptions of known documents, such as WO 2017/047745.

### EXAMPLES

Examples of the disclosure will now be described; however, the disclosure is not limited to the below-described Examples.

Hereinafter, unless otherwise specified, "room temperature" means 25°C.

Hereinafter, the purity (%) of a polythiol component A1 in a polythiol composition means a ratio (% by area) of a peak area of the polythiol component A1 with respect to a total peak area of compounds contained in the polythiol composition, which ratio is determined by high-performance liquid chromatography under the above-described measurement conditions A.

### [Comparative Example 1]

### <Production of Polythiol Composition X1 (for Comparison)>

A polythiol composition X1 containing 4-mercaptomethyl-1,8-dimercapto-3,6-dithiaoctane (i.e., polythiol component A1) as a main component was produced in accordance with the production method of Example A-1 of WO 2014/027427.

### <High-Performance Liquid Chromatography (HPLC) Measurement of Polythiol Composition X1>

For the polythiol composition X1, high-performance liquid chromatography (HPLC) measurement was performed under the above-described measurement conditions A. As a column, YMC-Pack ODS-A (manufactured by YMC Co., Ltd.) was used.

Further, liquid-chromatography mass spectrometry was performed under the above-described measurement conditions B.

Table 1 shows, as determined by the above-described HPLC of the polythiol composition X1:
the peak area ratio (more specifically, the peak area ratio with respect to a total peak area of the compounds contained in the polythiol composition; the same applies hereinafter) of the polythiol component A1;
the peak area ratio of a compound C1;
the peak area ratio of a compound C2;
the peak area ratio of a compound (XB); and
the peak area ratio of a compound (XC).

It is noted here that:
the polythiol component A1 is 4-mercaptomethyl-1,8-dimercapto-3,6-dithiaoctane;
the compound C1 is a polythiol compound that has a retention time of from 32.0 minutes to 35.0 minutes and a molecular weight of 426;
the compound C2 is a polythiol compound that has a retention time of from 16.0 minutes to 19.0 minutes,
the compound (XB) is a compound obtained by substituting at least one of mercapto groups in the polythiol component A1 with a group represented by Formula (N1); and
the compound (XC) is a compound obtained by substituting at least one of mercapto groups in the polythiol component A1 with a hydroxy group.

The structures of the respective compounds were estimated based on the results of the high-performance liquid chromatography (HPLC) measurement performed under the above-described measurement conditions A and the liquid-chromatography mass spectrometry performed under the above-described measurement conditions B, as well as the structure of the polythiol component A1 used as the main component.

The compound C1 in this Comparative Example 1 and the below-described Example 1 is presumed to be the above-described compound (C1-1).

### <Production of Polymerizable Composition and Formed Body>

In a flask equipped with a stirrer,
dibutyl tin dichloride (100 ppm by mass with respect to a total amount of the below-described polyisocyanate compound and the below-described polythiol composition X1) as a polymerization catalyst,
ZELEC-UN (manufactured by Stepan Company; acidic phosphoric acid ester) (1,000 ppm by mass with respect to a total amount of the below-described polyisocyanate compound and the below-described polythiol composition X1) as a mold release agent,
m-xylylene diisocyanate (XDI) (52 parts by mass) as a polyisocyanate compound, and the above-prepared polythiol composition X1 (48 parts by mass)
were added and mixed with stirring for 1 hour at room temperature (25°C) to obtain a polymerizable composition in the form of a transparent homogeneous solution.

Next, the thus obtained polymerizable composition was vacuum-filtered through a polytetrafluoroethylene (PTFE) filter, and then thoroughly degassed under a reduced pressure of 600 Pa until foaming was no longer observed. The thus degassed polymerizable composition was injected between a pair of glass molds immobilized by a tape, and then, this pair of glass molds was placed in an oven, after which the oven internal temperature was set at 10°C. Subsequently, the oven internal temperature was raised from 10°C to 120°C over a period of 38 hours. By this process, monomers (the polyisocyanate compound and the polythiol composition) contained in the degassed polymerizable composition were polymerized to form a formed body containing a thiourethane resin R1 (i.e., a cured product of the polymerizable composition) between the pair of glass molds.

Thereafter, the inside of the oven was cooled, and the pair of glass molds was subsequently taken out of the oven, after which the formed body was obtained by removing it from the pair of glass molds.

### <Lens Production>

A lens was produced by cutting the thus obtained formed body.

### <Measurement and Evaluation of Lens>

For the thus obtained lens, the refractive index, the heat resistance (glass transition temperature (Tg)), and the specific gravity d were measured by the below-described respective methods.

The results thereof are shown in Table 1.
- Refractive index (ne): using a Pulfrich refractometer KPR-30 manufactured by Shimadzu Corporation, the refractive index (ne) was measured at 20°C and a wavelength of 546.1 nm (mercury e-line).
- Heat resistance (Tg): using a thermomechanical analyzer TMA-60 manufactured by Shimadzu Corporation, the glass transition temperature (Tg) was measured by a TMA penetration method (load: 50 g, pin tip: 0.5 mmϕ, heating rate: 10°C/min), and the measured value was used as an index of the heat resistance. A higher Tg indicates superior heat resistance.
- Specific gravity d: measured at 20°C by Archimedes' method.

### [Example 1]

### <Production of Polythiol Composition>

### -Preparation of Thiourethane Resin Powder-

A thiourethane resin powder R1 (i.e., powder containing the thiourethane resin R1) was obtained by collecting cut powder generated during the lens production (i.e., cutting) in Comparative Example 1.

### -Decomposition Reaction of Thiourethane Resin R1 by Monoethanolamine-

The thiourethane resin powder R1 (200 g) was charged into a 1,000-mL flask equipped with a condenser, and monoethanolamine (manufactured by FUJIFILM Wako Pure Chemical Corporation) (81.0 g; 1.326 mol) and toluene (385.6 g) were added thereto, followed by heating with stirring at 90°C for 5 hours, whereby a reaction mixture containing a polythiol composition was obtained (the above operations are hereinafter referred to as "reaction step").

### -Separation of Polythiol Composition-

The reaction mixture obtained by the reaction step was subjected to the separation step including extraction and the like, whereby the polythiol composition was separated from the reaction mixture. The details of this process will now be described.

The reaction mixture obtained in the reaction step was cooled to 60°C, and solid was subsequently removed by filtration. To the resulting filtrate, 144.3 g of a 31% aqueous sodium hydroxide solution was added, followed by stirring at 25°C. To the resultant, 150 g of water was added to extract soluble components, and the resulting water extract was washed with 50 g of toluene at 25°C, after which 200 g of toluene and then 150 g of 35% hydrochloric acid were added, and the resultant was stirring at 40°C. Soluble components were extracted with toluene to obtain a toluene extract. This toluene extract was washed with 160 g of water at 40°C and then with 160 g of 0.1% aqueous ammonia at 40°C, and further washed twice with 160 g of water at 40°C, whereby a toluene solution of the polythiol composition was obtained.

From the thus obtained toluene solution, toluene was distilled away using a rotary evaporator. The thus obtained mixture was sequentially subjected to removal of a low-boiling-point component by a vacuum pump and filtration through a 1-micron PTFE membrane filter in this order, whereby 86.2 g of a polythiol composition containing 4-mercaptomethyl-1,8-dimercapto-3,6-dithiaoctane (i.e., polythiol component A1) as a main component was obtained (the above operations are hereinafter referred to as "separation step").

### <High-Performance Liquid Chromatography (HPLC) Measurement of Polythiol Composition>

For the polythiol composition obtained in Example 1, HPLC measurement was performed in the same manner as in Comparative Example 1.

The results thereof are shown in Table 1.

### <Production of Polymerizable Composition, Formed Body, and Lens>

A polymerizable composition, a formed body, and a lens were produced in the same manner as in Comparative Example 1, except that the polythiol composition X1 used in Comparative Example 1 was changed to the polythiol composition obtained in Example 1.

### <Measurement and Evaluation of Lens>

For the lens obtained in Example 1, the refractive index, the heat resistance, and the specific gravity d were measured in the same manner as in Comparative Example 1.

The results thereof are shown in Table 1.

**[Table 1]**

| | | Comparative Example 1 | Example 1 |
|---|---|---|---|
| HPLC Results of polythiol composition | Peak area ratio of polythiol component A1 | 91.8 | 93.5 |
| | Peak area ratio of compound C1 | 0.41 | 1.29 |
| | Peak area ratio of compound C2 | 2.37 | 2.93 |
| | Peak area ratio of compound (XB) | 0.53 | 0.04 |
| | Peak area ratio of compound (XC) | 2.62 | 0.03 |
| Lens evaluation results | Refractive index (ne) | 1.665 | 1.665 |
| | Tg (°C) | 85 | 88 |
| | Specific gravity d | 1.35 | 1.36 |

As shown in Table 1, in Example 1 where the polythiol composition which contained the compound C1 having a retention time of from 32.0 minutes to 35.0 minutes and exhibiting a peak area of 0.50 or more with respect to a total peak area of 100 of the compounds contained in the polythiol composition in the above-described high-performance liquid chromatography measurement was used, a thiourethane resin having superior heat resistance (i.e., a lens having a higher Tg) was produced as compared to Comparative Example 1 where the polythiol composition in which a peak area of the compound C1 is less than 0.50 was used.

A mixture obtained by mixing the polythiol composition of Comparative Example 1 and the polythiol composition of Example 1 is also useful.

For example, in a mixture obtained by mixing 90 parts by mass of the polythiol composition of Comparative Example 1 and 10 parts by mass of the polythiol composition of Example 1, the compound C1 has a peak area of 0.50.

### [Reference Example 1]

### <Preparation of Polythiol Composition X2>

In accordance with the production method of Example C-1 of WO 2014/027428, a polythiol composition X2 containing a combination of 4,8-dimercaptomethyl-1,1 1-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane (i.e., polythiol component A2) as a main component was prepared.

### <Production of Polymerizable Composition and Formed Body>

In a flask equipped with a stirrer,
dimethyl tin dichloride (trade name: NESTIN P, manufactured by The Honjo Chemical Corporation) (100 ppm by mass with respect to a total amount of the below-described polyisocyanate compound and the below-described polythiol composition X2) as a polymerization catalyst,
ZELEC-UN (manufactured by Stepan Company; acidic phosphoric acid ester) (1,000 ppm by mass with respect to a total amount of the below-described polyisocyanate compound and the below-described polythiol composition X2) as a mold release agent,
*m*-xylylene diisocyanate (XDI) (50.8 parts by mass) as a polyisocyanate compound, and the above-prepared polythiol composition X2 (49.2 parts by mass)
were added and mixed with stirring for 1 hour at room temperature to obtain a polymerizable composition in the form of a transparent homogeneous solution.

Next, the thus obtained polymerizable composition was vacuum-filtered through a polytetrafluoroethylene (PTFE) filter, and then thoroughly degassed under a reduced pressure of 600 Pa until foaming was no longer observed. The thus degassed polymerizable composition was injected between a pair of glass molds immobilized by a tape, and then, this pair of glass molds was placed in an oven, after which the oven internal temperature was set at 25°C. Subsequently, the oven internal temperature was raised from 25°C to 120°C over a period of 24 hours. By this process, monomers (the polyisocyanate compound and the polythiol composition) contained in the degassed polymerizable composition were polymerized to form a formed body containing a thiourethane resin R2 (i.e., a cured product of the polymerizable composition) between the pair of glass molds.

Thereafter, the inside of the oven was cooled, and the pair of glass molds was subsequently taken out of the oven, after which the formed body of Reference Example 1 was obtained by removing it from the pair of glass molds.

### <Lens Production>

A lens of Reference Example 1 was produced by cutting the thus obtained formed body of Reference Example 1.

### [Example 2]

### <Production of Polythiol Composition>

A thiourethane resin powder R2 (i.e., powder containing the thiourethane resin R2) was obtained by collecting cut powder generated during the lens production (i.e., cutting) in Comparative Example 1.

### -Decomposition Reaction of Thiourethane Resin R2 by Monoethanolamine-

The whole amount of the thiourethane resin powder R2 (50 g) was charged into a 500-mL flask equipped with a condenser, and monoethanolamine (manufactured by FUJIFILM Wako Pure Chemical Corporation) (19.75 g; 0.323 mol) and 180.25 g of xylene were added thereto, followed by heating with stirring at 100°C for 5 hours, whereby a reaction mixture containing a polythiol composition was obtained (the above operations are hereinafter referred to as "reaction step").

### -Separation of Polythiol Composition-

The thus obtained reaction mixture was cooled to 30°C, and solid was subsequently removed by filtration. The resulting filtrate was washed twice with 20 g of 35% hydrochloric acid to remove excess amine from the filtrate. The thus obtained liquid was washed twice with 50 g of water to obtain a xylene solution of the polythiol composition.

From the thus obtained xylene solution, xylene was distilled away using a rotary evaporator. The thus obtained mixture was sequentially subjected to removal of a low-boiling-point component by a vacuum pump and filtration through a 1-micron PTFE membrane filter in this order, whereby 19.49 g of a polythiol composition containing a combination of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane (i.e., polythiol component A2) as a main component was obtained (the above operations are hereinafter referred to as "separation step").

### <High-Performance Liquid Chromatography (HPLC) Measurement of Polythiol Composition>

For the thus obtained polythiol composition, high-performance liquid chromatography (HPLC) measurement was performed under the above-described measurement conditions A. As a column, YMC-Pack ODS-A (manufactured by YMC Co., Ltd.) was used.

Further, liquid-chromatography mass spectrometry was performed under the above-described measurement conditions B.

Table 2 shows, as determined by the above-described HPLC of the polythiol composition:
the peak area ratio (more specifically, the peak area ratio with respect to a total peak area of the compounds contained in the polythiol composition; the same applies hereinafter) of the polythiol component A2;
the peak area ratio of a compound C1;
the peak area ratio of a compound C2;
the peak area ratio of a compound (XB); and
the peak area ratio of a compound (XC).

It is noted here that:
the polythiol component A2 is a combination of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane;
the compound C1 is a polythiol compound that has a retention time of from 32.0 minutes to 35.0 minutes and a molecular weight of 426;
the compound C2 is a polythiol compound that has a retention time of from 16.0 minutes to 19.0 minutes,
the compound (XB) is a compound obtained by substituting at least one of mercapto groups in the polythiol component A2 with a group represented by Formula (N1); and
the compound (XC) is a compound obtained by substituting at least one of mercapto groups in the polythiol component A2 with a hydroxy group.

The structures of the respective compounds were estimated based on the results of the high-performance liquid chromatography (HPLC) measurement performed under the above-described measurement conditions A and the liquid-chromatography mass spectrometry performed under the above-described measurement conditions B, as well as the structure of the polythiol component A2 used as the main component.

The compound C1 in this Example 2 is presumed to be at least one selected from the group consisting of the above-described compounds (C1-2a), (C1-2b), (C1-2c), and (C1-2d).

### <Production of Polymerizable Composition, Formed Body, and Lens>

A polymerizable composition, a formed body, and a lens were produced in the same manner as in Reference Example 1, except that the polythiol composition X2 used in Reference Example 1 was changed to the polythiol composition obtained in Example 2.

### <Measurement and Evaluation of Lens>

For the lens obtained in Example 2, the refractive index, the heat resistance (Tg), and the specific gravity d were measured in the same manner as in Example 1.

The results thereof are shown in Table 2.

**[Table 2]**

| | | Example 2 |
|---|---|---|
| HPLC Results of polythiol composition | Peak area ratio of polythiol component A2 | 83.9 |
| | Peak area ratio of compound C 1 | 2.67 |
| | Peak area ratio of compound C2 | 1.41 |
| | Peak area ratio of compound (XB) | 0.00 |
| | Peak area ratio of compound (XC) | 0.00 |
| Lens evaluation results | Refractive index (ne) | 1.669 |
| | Tg (°C) | 102 |
| | Specific gravity d | 1.37 |

As shown in Table 2, a thiourethane resin having excellent heat resistance (i.e., a lens having a high Tg) was produced in Example 2 where the polythiol composition which contained the compound C1 having a retention time of from 32.0 minutes to 35.0 minutes and exhibiting a peak area of 0.50 or more with respect to a total peak area of 100 of the compounds contained in the polythiol composition in the above-described high-performance liquid chromatography measurement was used.

The disclosure of Japanese Patent Application No. 2021-126033 filed on July 30, 2021 is hereby incorporated by reference in its entirety.

All the documents, patent applications and technical standards that are described in the present specification are hereby incorporated by reference to the same extent as if each individual document, patent application or technical standard is concretely and individually described to be incorporated by reference.

## Claims

1. A polythiol composition, comprising a polythiol compound,
wherein:
the polythiol composition comprises a compound C1 that has a retention time of from 32.0 minutes to 35.0 minutes in a high-performance liquid chromatography measurement performed under the following measurement conditions A, and
in the high-performance liquid chromatography measurement, the compound C1 has a peak area of 0.50 or more with respect to a total peak area of 100 of compounds contained in the polythiol composition:
Measurement Conditions A
YMC-Pack (registered trademark) ODS-A manufactured by YMC., Co., Ltd. (particle size S: 5 µm, pore diameter: 12 nm, column shape: Φ6 mm × 150 mm) is used as a column;
a mixed solution of acetonitrile/0.01-mol/L aqueous potassium dihydrogen phosphate solution = 60/40 (vol/vol) is used as a mobile phase;
a mixed solution of 160 mg of the polythiol composition and 10 mL of acetonitrile is used as a measurement solution;
a UV detector having a measurement wavelength of 230 nm is used as a detector;
the column temperature is set at 40°C;
the flow rate is set at 1.0 mL/min; and
the injection amount is set at 2 µL.

2. The polythiol composition according to claim 1, wherein the compound C1 has a molecular weight of 426.

3. The polythiol composition according to claim 1 or 2, wherein the compound C1 is a polythiol compound.

4. The polythiol composition according to any one of claims 1 to 3,
wherein:
the polythiol composition further comprises a compound C2 that has a retention time of from 16.0 minutes to 19.0 minutes in the high-performance liquid chromatography measurement, and
in the high-performance liquid chromatography measurement, the compound C2 has a peak area of 1.39 or more with respect to a total peak area of 100 of compounds contained in the polythiol composition.

5. The polythiol composition according to any one of claims 1 to 4,
wherein:
the polythiol composition further comprises, as a main component, a polythiol compound (XA) comprising three or more mercapto groups,
wherein:
in the high-performance liquid chromatography measurement, a peak area of a compound (XB), the compound (XB) being obtained by substituting at least one of the three or more mercapto groups in the polythiol compound (XA) with a group represented by the following Formula (N1), is less than 1.10 with respect to a total peak area of 100 of compounds contained in the polythiol composition,
and wherein:
in the high-performance liquid chromatography measurement, a peak area of a compound (XC), the compound (XC) being obtained by substituting at least one of the three or more mercapto groups in the polythiol compound (XA) with a hydroxy group, is less than 2.40 with respect to a total peak area of 100 of compounds contained in the polythiol composition: 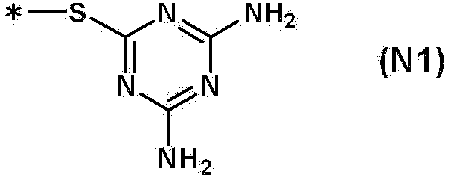
wherein, in Formula (N1), * represents a binding position.

6. The polythiol composition according to claim 5, wherein the polythiol compound (XA) is at least one selected from the group consisting of 4-mercaptomethyl-1,8-dimercapto-3,6-dithiaoctane, 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.

7. A polymerizable composition for an optical material, comprising:
a polyiso(thio)cyanate compound; and
the polythiol composition according to any one of claims 1 to 6.

8. The polymerizable composition for an optical material according to claim 7, wherein the polyiso(thio)cyanate compound comprises at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.

9. A resin, which is a cured product of the polymerizable composition for an optical material according to claim 7 or 8.

10. A formed body, comprising the resin according to claim 9.

11. An optical material, comprising the resin according to claim 9.

12. A lens, comprising the resin according to claim 9.
